# EUROPEAN PATENT APPLICATION

(11) **EP 4 603 009 A1**
(43) Date of publication of application: **20.08.2025**
(21) Application number: 25152533.3
(22) Date of filing: 17.01.2025
(51) Int. Cl.: A61B 5/03, A61M 5/168, G01L 19/14

(54) **PRESSURE SENSOR, ASSOCIATED METHOD, AND ASSOCIATED MEDICATION DELIVERY DEVICE**

(30) Priority: 12.02.2024 IN 202411009215
(71) Applicant: Honeywell International Inc., Charlotte, NC 28202 (US)
(72) Inventor: MAUT, Sandeep Laxman, Charlotte, 28202 (US); HM, Manjunatha, Charlotte, 28202 (US); MURALI, HariPrasad Padubidri, Charlotte, 28202 (US)
(74) Representative: Haseltine Lake Kempner LLP

(57) **Abstract**

In accordance with various embodiments of the present disclosure, a pressure sensor is provided. In some embodiments, the pressure sensor comprises a circuit board, a sensing element eccentrically positioned on the circuit board, a cover portion mounted to the circuit board and comprising a columnar portion defining a port, and a column of bio-compatible gel disposed within the port. The columnar portion has a distal open end and a proximal open end that is adhered to the circuit board and that surrounds the sensing element such that the columnar portion is eccentrically positioned relative to the circuit board. The columnar portion is connected to a reservoir containing a fluid, and the fluid is exposed to the gel column via the distal open end and such that a pressure of the fluid is translated by the gel column to the sensing element such that the sensing element detects the fluid pressure.

## Description

### FIELD OF THE INVENTION

Example embodiments of the present disclosure relate generally to pressure sensors and, more particularly, to pressure sensors for use with medication delivery devices.

### BACKGROUND

Hundreds of millions of people have diabetes. Many of them rely on daily insulin injections to control their blood sugar levels. Increasingly, insulin users rely on insulin pumps to deliver insulin into their bodies. One type of insulin pump is the insulin patch pump. An insulin patch pump is a body mounted device with an internal reservoir holding enough insulin for 1-3 days and a small catheter needle connected to the reservoir by a flow tube. The needle delivers insulin subcutaneously to the user.

One common problem with insulin patch pumps is that the flow of insulin may be reduced or stopped because, for example, of an obstruction of the needle or a bend or kink in the flow tube. Failure to deliver the correct dose of insulin may eventually lead to diabetic ketoacidosis (DKA), a serious condition that can lead to diabetic coma or even death.

Through applied effort, ingenuity, and innovation, many of these identified problems have been solved by developing solutions that are included in embodiments of the present disclosure, many examples of which are described in detail herein.

### BRIEF SUMMARY

Various embodiments described herein relate to pressure sensors, methods of measuring the pressure of fluid in a reservoir, and medication delivery devices.

In accordance with various embodiments of the present disclosure, a pressure sensor is provided. In some embodiments, the pressure sensor comprises a circuit board, a sensing element eccentrically positioned on a first major side of the circuit board, a cover portion mounted to the first major side of the circuit board and comprising a columnar portion defining a port, and a column of bio-compatible gel disposed within the port. The columnar portion has a distal open end and a proximal open end that is adhered to the circuit board and that surrounds the sensing element such that the columnar portion is eccentrically positioned relative to the first major side of the circuit board. The columnar portion is adapted to be connected to a reservoir containing a fluid such that the fluid is exposed to the column of bio-compatible gel via the distal open end and such that a pressure of the fluid in the reservoir is translated by the column of bio-compatible gel to the sensing element such that the sensing element is adapted to detect the pressure of the fluid in the reservoir.

In some embodiments, the columnar portion is positioned relative to the first major side of the circuit board such that the columnar portion is offset a first distance from a center of the first major side of the circuit board in a first direction and a second distance from the center of the first major side of the circuit board in a second direction.

In some embodiments, the first distance and the second distance are substantially the same and the first direction and the second direction are substantially perpendicular.

In some embodiments, an inner surface of the port defines an angle change line, a distal portion of the inner surface of the port between the angle change line and the distal open end is cylindrical, and a proximal portion of the inner surface of the port between the angle change line and the proximal open end is angled inward.

In some embodiments, the proximal portion of the inner surface of the port is angled inward at less than ten degrees.

In some embodiments, the angle change line is located a distance from the circuit board that is equal to or greater than a distance from the circuit board of a furthest away portion of a wirebond attached to the sensing element.

In some embodiments, an outer surface of the columnar portion of the cover portion comprises a distal circumferential shoulder adjacent the distal open end and a proximal circumferential shoulder spaced apart therefrom such that a circumferential groove is defined therebetween. The circumferential groove is adapted to receive an O-ring to seal a connection between the reservoir and the columnar portion of the cover portion.

In some embodiments, the pressure sensor further comprises an application specific integrated circuit (ASIC) positioned on the first major side of the circuit board. The cover portion further comprises an extended portion defining a chamber separated from the port. The ASIC is at least partially enclosed within the chamber of the extended portion. The ASIC receives a piezoelectric current from the sensing element which is proportional to the pressure of the fluid in the reservoir. The ASIC uses the received piezoelectric current to compute the pressure of the fluid in the reservoir and/or a change in the pressure of the fluid in the reservoir.

In some embodiments, the ASIC is adapted to use the computed pressure of the fluid in the reservoir and/or the change in the pressure of the fluid in the reservoir to detect a reduced or stopped flow of the fluid from the reservoir.

In some embodiments, the cover portion is adhered to the first major side of the circuit board using an adhesive. One or more surfaces of the cover portion in contact with the adhesive comprise one or more bumps projecting therefrom and have a rougher surface texture than other surfaces of the cover portion.

In accordance with various embodiments of the present disclosure, a method for measuring a pressure of a fluid in a reservoir is provided. In some embodiments, the method comprises connecting a pressure sensor to the reservoir. The pressure sensor comprises a circuit board, a sensing element eccentrically positioned on a first major side of the circuit board, a cover portion mounted to the first major side of the circuit board and comprising a columnar portion defining a port, and a column of bio-compatible gel disposed within the port. The columnar portion has a distal open end and a proximal open end that is adhered to the circuit board and that surrounds the sensing element such that the columnar portion is eccentrically positioned relative to the first major side of the circuit board. The columnar portion of the pressure sensor is connected to the reservoir such that the fluid in the reservoir is exposed to the column of bio-compatible gel via the distal open end. The pressure of the fluid in the reservoir is translated by the column of bio-compatible gel to the sensing element such that the sensing element detects the pressure of the fluid in the reservoir.

In accordance with various embodiments of the present disclosure, a medication delivery device is provided. In some embodiments, the medication delivery device comprises a reservoir containing a fluid medication and a pressure sensor attached to the reservoir. The pressure sensor comprises a circuit board, a sensing element eccentrically positioned on a first major side of the circuit board, a cover portion mounted to the first major side of the circuit board and comprising a columnar portion defining a port, and a column of bio-compatible gel disposed within the port. The columnar portion has a distal open end and a proximal open end that is adhered to the circuit board and that surrounds the sensing element such that the columnar portion is eccentrically positioned relative to the first major side of the circuit board. The columnar portion of the pressure sensor is connected to the reservoir such that the fluid in the reservoir is exposed to the column of bio-compatible gel via the distal open end. The pressure of the fluid in the reservoir is translated by the column of bio-compatible gel to the sensing element such that the sensing element detects the pressure of the fluid in the reservoir.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a perspective view of an example pressure sensor, in accordance with example embodiments of the present disclosure;
FIG. 2 is an exploded perspective view of the example pressure sensor of FIG. 1;
FIG. 3 is a top view of the example pressure sensor of FIG. 1;
FIG. 4 is a sectional perspective view of the example pressure sensor of FIG. 1 along line A-A;
FIGS. 5 and 6 are section side views of the example pressure sensor of FIG. 1 along line A-A;
FIG. 7 is a side view of a cover portion of the example pressure sensor of FIG. 1;
FIG. 8 is a bottom perspective view of the cover portion of the example pressure sensor of FIG. 1; and
FIG. 9 is a sectional side view of an example medication delivery device using the example pressure sensor of FIG. 1, in accordance with example embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

Various embodiments of the present disclosure provide pressure sensors, methods of measuring the pressure of fluid in a reservoir, and medication delivery devices. Pressure sensors of various embodiments of the present disclosure may be used in any suitable device or application, such as any suitable medication delivery device, including, but not limited to, insulin patch pumps. Insulin patch pumps are typically used for 1-3 days and then discarded, and therefore are considered to be disposable. Pressure sensors of various embodiments of the present disclosure may be used in such disposable insulin patch pumps and may therefore also be considered to be disposable.

In various embodiments of the present disclosure, a pressure sensor comprises a sensing element (e.g., sense die, transducer, and/or the like) that is eccentrically positioned (i.e., off centered) on a circuit board. In various embodiments, the pressure sensor comprises a cover portion mounted to the circuit board and comprising a columnar portion defining a port. In various embodiments, a column of bio-compatible gel (e.g., silicone gel) is disposed within the port. In various embodiments, a proximal open end of the columnar portion surrounds the sensing element such that the columnar portion is eccentrically positioned relative to the circuit board. In various embodiments, this eccentric positioning of the columnar portion and the sensing element enables the sensing element to be isolated from other electronic components of the pressure sensor (such as the application specific integrated circuit, described below), thereby preventing the bio-compatible gel from contacting and potentially damaging the other electronic components and/or eliminating the need to protect the other electronic components (such as by the use of potting). In various embodiments, this eccentric positioning of the columnar portion and the sensing element also enables a reduction in mechanical stress on the sensing element during assembly of the insulin patch pump.

In various embodiments, the columnar portion is connected to a reservoir (such as of a medication delivery device, such as an insulin patch pump) containing a fluid (such as insulin). In various embodiments, the fluid in the reservoir is exposed to the column of bio-compatible gel via the port such that a pressure of the fluid in the reservoir is translated by the column of bio-compatible gel to the sensing element. In this regard, the sensing element is able to detect the pressure of the fluid in the reservoir.

In various embodiments, the pressure sensor further comprises an application specific integrated circuit (ASIC) positioned on the circuit board. In various embodiments, the ASIC receives a piezoelectric current from the sensing element which is proportional to the pressure of the fluid in the reservoir. In various embodiments, the ASIC uses the received piezoelectric current to compute the pressure of the fluid in the reservoir and/or a change in the pressure of the fluid in the reservoir. In various embodiments, an occlusion or other interruption/reduction in the flow of fluid from the reservoir causes a change in pressure of the fluid.

In various embodiments, the pressure sensor uses the detected pressure of the fluid in the reservoir and/or a change in the pressure of the fluid in the reservoir to detect a reduced or stopped flow of the fluid from the reservoir. In some embodiments, the pressure sensor detects a reduced or stopped flow of the fluid from the reservoir based on the pressure of the fluid exceeding a predetermined threshold. In other embodiments, the pressure sensor detects a reduced or stopped flow of the fluid from the reservoir based on the pressure of the fluid unexpectedly increasing or unexpectedly decreasing and/or increasing or decreasing by an unexpected amount.

In various embodiments, a lower portion of the port is angled inward (this inward angle may be termed a draft angle). In various embodiments, this inward angling of the lower portion of the port helps in channelizing the gel column onto the center of the sensing element, thus improving the sensitivity of the sensor by reducing the volume of gel over the sensing element. In various embodiments, reducing the volume of gel over the sensing element also enables a substantial increase in the ability of the pressure sensor to withstand burst pressure (e.g., increasing from withstanding about three times burst pressure to withstanding about ten times burst pressure). In some embodiments, the angled lower portion of the port extends above the wirebonds attached to the sensing element.

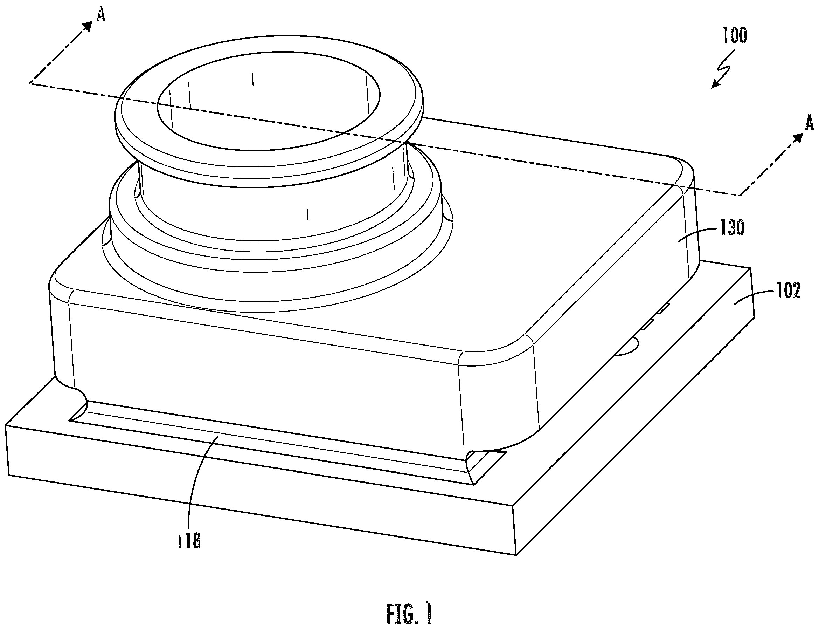

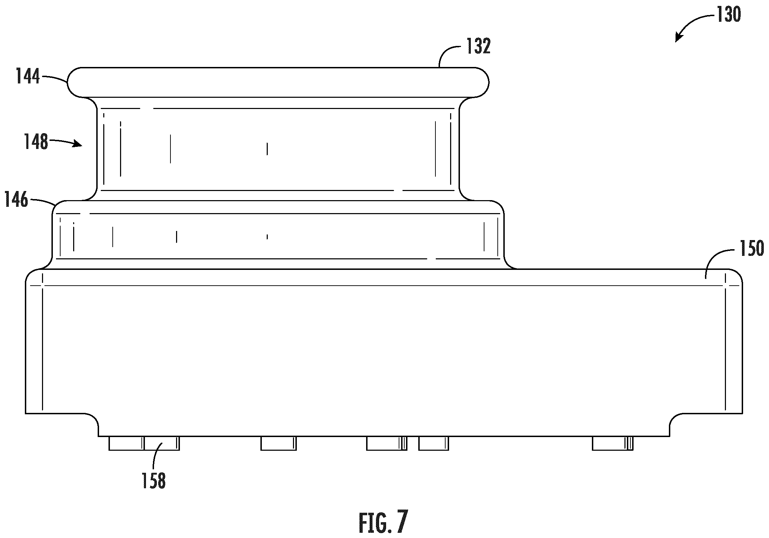

Referring now to the figures, FIGS. 1-8 illustrate an example pressure sensor, such as may be used with a medication delivery device such as an insulin pump patch, in accordance with example embodiments of the present disclosure. The pressure sensor 100 of FIGS. 1-8 comprises a circuit board 102 and a cover portion 130. In the illustrated embodiment, the cover portion 130 is adhered to the circuit board 102 using any suitable adhesive (as described further below).

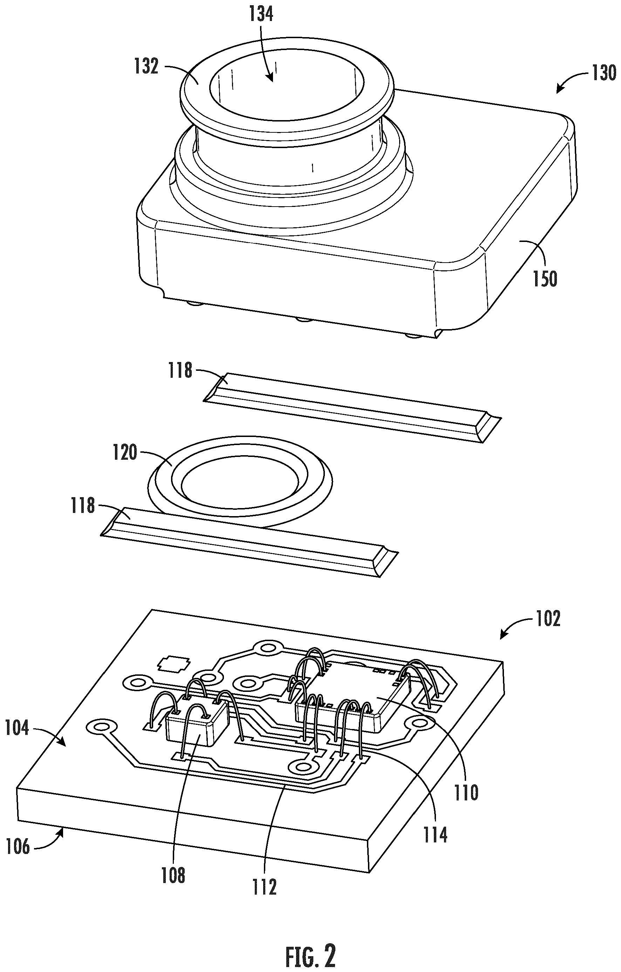

As best seen in FIG. 2, pressure sensor 100 of the illustrated embodiment comprises a sensing element 108 (such as a sense die) and an ASIC 110 (or any other suitable special purpose or general-purpose controller) mounted on a first major surface 104 of the circuit board 102 (the circuit board 102 also comprises a second major surface 106 opposite the first major surface). Electrical connections between the sensing element 108 and the ASIC 110 are made via surface conductors 112 and wirebonds 114. The cover portion 130 acts as a housing to protect the sensing element 108, the ASIC 110, and the wirebonds 114.

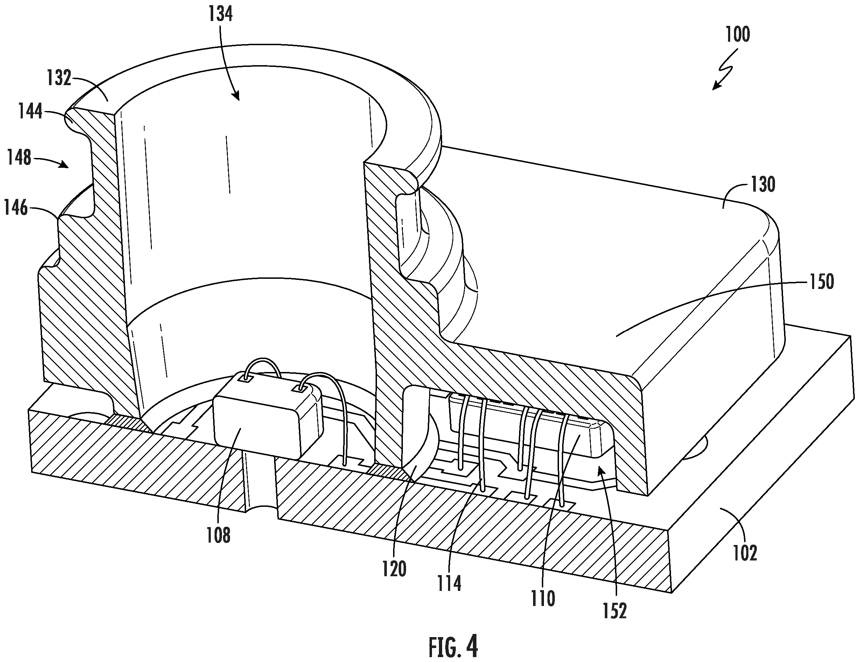

In the illustrated embodiment, the cover portion 130 comprises a columnar portion 132 and an extended portion 150. The columnar portion 132 defines a generally cylindrical port 134 (but with an angled portion in some embodiments, as described herein). The extended portion 150 defines a chamber 152, as seen in FIGS. 4-6 and 8.

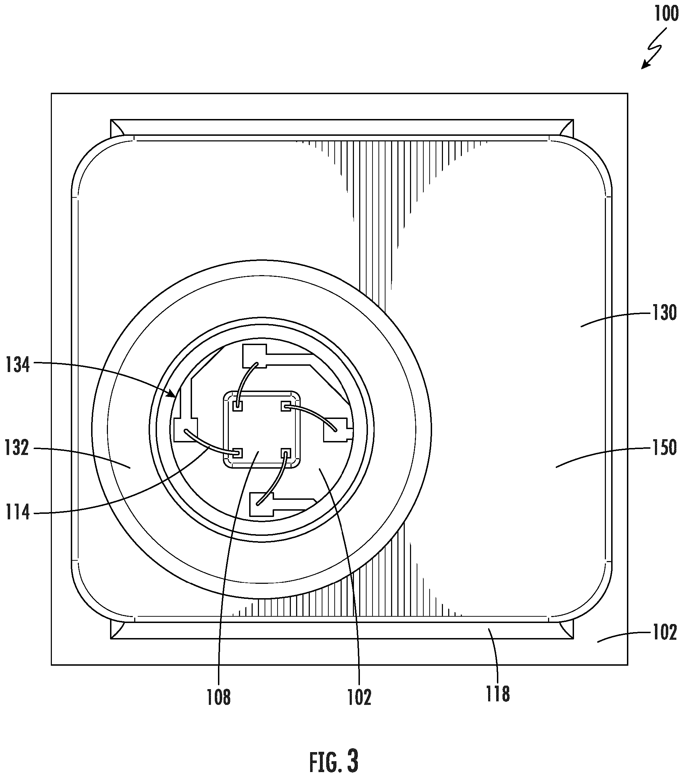

In the illustrated embodiment, the columnar portion 132 of the cover portion 130 and the sensing element 108 are both eccentrically positioned (i.e., off center) relative to the centers of the circuit board 102 and of the cover portion 130 (in various embodiments, the centers of the circuit board 102 and of the cover portion 130 are substantially aligned), such that the columnar portion 132 and the sensing element 108 are generally aligned. This alignment is seen in the top view of FIG. 3, in which the sensing element 108 is seen through and substantially centered on the port 134. In some embodiments, the columnar portion 132 is offset from the centers of the circuit board 102 and the cover portion 130 in a first (e.g., X) direction by a first distance and in a second (e.g., Y) direction by a second distance. In some embodiments, the first distance and the second distance are substantially the same and the first direction and the second direction are substantially perpendicular. In some embodiments, the offset is such that two adjacent edges of the cover portion are tangential (or nearly tangential) to the columnar portion.

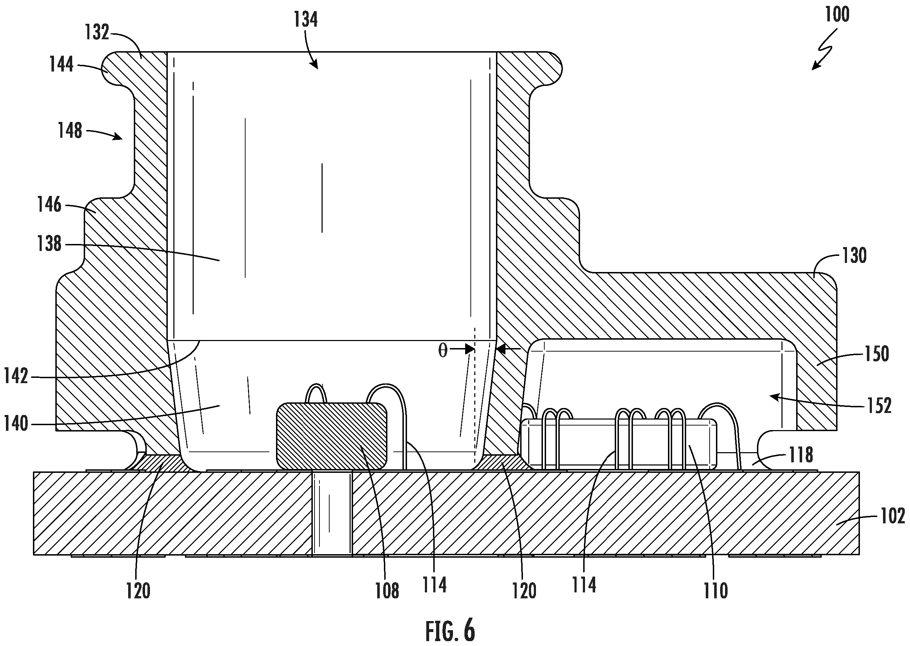

The columnar portion 132 has an upper or distal (relative to the circuit board) portion 138 that is generally cylindrical (i.e., having generally vertical walls) and a lower or proximal (relative to the circuit board) portion 140 that is angled inward (i.e., toward a longitudinal axis of the columnar portion 132). The dividing line 142 between the distal portion 138 and the proximal portion 140 may be termed an angle change line. In various embodiments, this draft angle (labeled θ in FIG. 6) may be less than about ten degrees, and may be, for example, about six to eight degrees. In some embodiments, as seen in FIG. 6, the angle change line 142 is above the wirebonds attached to the sensing element. This helps ensure that the sensing element remains secured to the circuit board when the gel is dispensed into the port and reduces or eliminates bubble accumulation which improves the sensitivity of the pressure sensor.

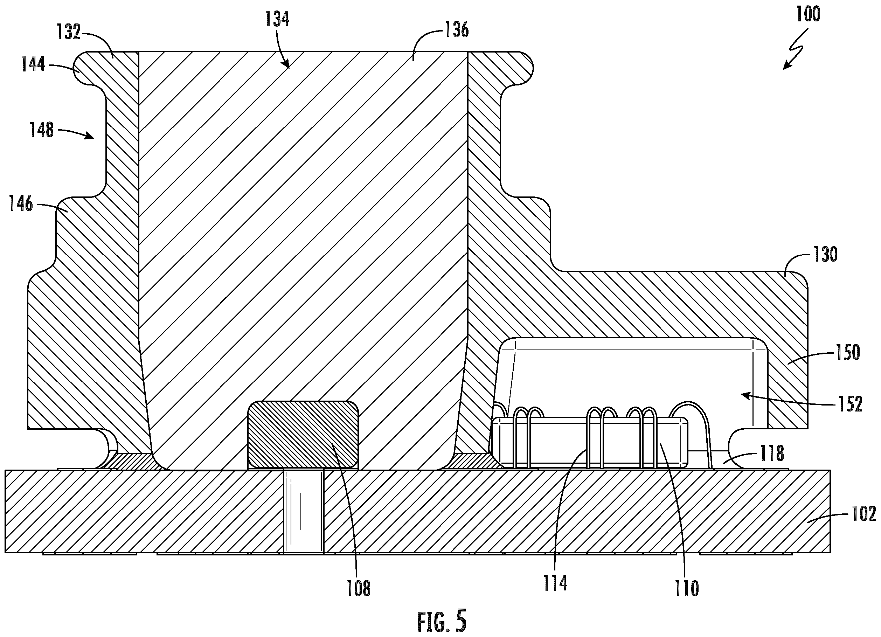

As seen in FIG. 5, the port 134 is filled with a column of bio-compatible gel 136 (the gel is omitted from the other figures for clarity). The column of bio-compatible gel is in contact with a fluid in the reservoir via the port 134 such that a pressure of the fluid in the reservoir is translated by the column of bio-compatible gel 136 to the sensing element 108. A bio-compatible gel, such as, e.g., silicone gel, is used so that there is no risk to the patient by the contact between the gel and the fluid medication.

The extended portion 150 defines a chamber 152, as seen in FIGS. 4-6 and 8. The ASIC 110 is at least partially enclosed within the chamber of the extended portion and therefore protected.

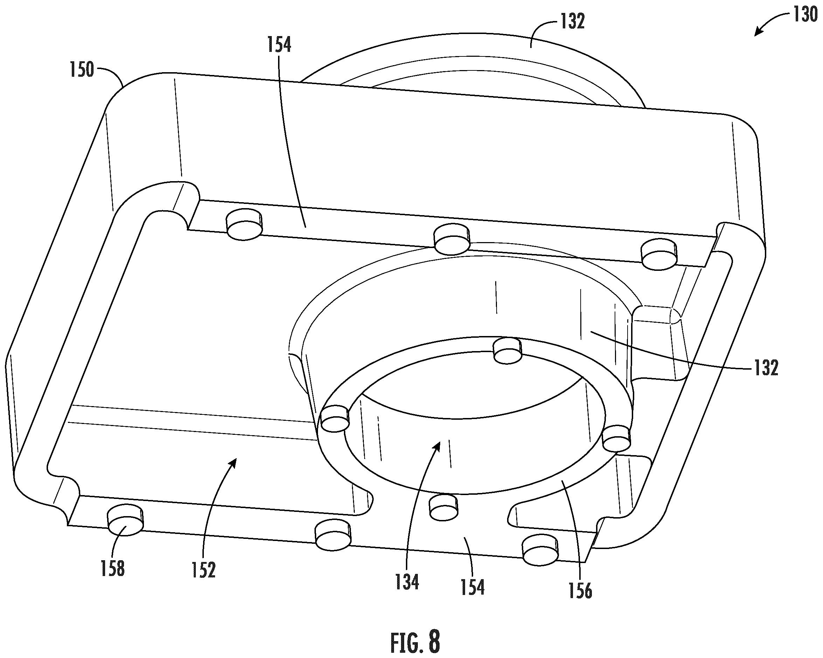

In the illustrated embodiment, the cover portion 130 is adhered to the circuit board 102 using two elongated strips of adhesive 118 that adhere to corresponding edge contact surfaces 154 and a circle of adhesive 120 that adheres to a circle contact surface 156 of the columnar portion 132 (the strips of adhesive 118 and the circle of adhesive 120 are seen in FIG. 2, while the edge contact surfaces 154 and the circle contact surface 156 are seen in FIG. 8). The contact between the circle of adhesive 120 (once cured) and circle contact surface 156 of the columnar portion 132 helps prevent the bio-compatible gel 136 from leaking from the port 134 and reaching the ASIC 110.

In the illustrated embodiment, the edge contact surfaces 154 and the circle contact surface 156 each have a plurality of bumps 158 projecting therefrom. When the adhesive is applied to the circuit board and the cover portion is pushed onto the adhesive, these bumps pierce through the wet adhesive bed and improve the adhesive strength. In various embodiments, the surface finish of the edge contact surfaces and the circle contact surface is rougher than the surface finish of other surfaces of the cover portion, which improves the adhesive strength and increases the shear strength.

In the illustrated embodiment, the outer surface of the upper or distal end of the columnar portion 132 comprises a distal circumferential shoulder 144 adjacent the distal open end and a proximal circumferential shoulder 146 spaced apart therefrom such that a circumferential groove 148 is defined therebetween. The circumferential groove 148 receives an O-ring to seal a connection between the reservoir and the columnar portion 132.

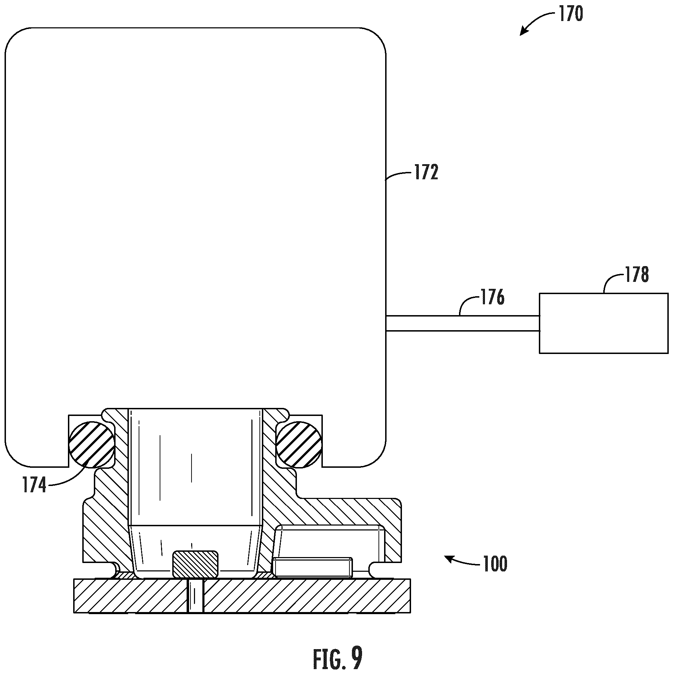

Referring now to FIG. 9, a sectional side view of an example medication delivery device using the example pressure sensor of example embodiments of the present disclosure is illustrated. The example medication delivery device 170 of FIG. 9 comprises a pressure sensor 100, as described above, connected to a reservoir 172 that would contain a fluid medication (not illustrated). An O-ring 174 at the connection of the pressure sensor 100 and the reservoir 172 helps prevent leakage at the connection. A flow tube 176 delivers the fluid medication from the reservoir 172 to a human body interface 178. In various embodiments, the human body interface 178 comprises a small catheter needle. In various embodiments, the fluid medication is pumped, drawn, or pushed out of the reservoir, using a pump, piston, or the like (not illustrated), as needed based on instructional programming as executed by a processor (not illustrated) of the medication delivery device.

In various embodiments, a disruption in the flow of the fluid medication from the reservoir, such as due to a kink in the flow tube 176 or a blockage of the catheter needle of the human body interface 178, would cause a pressure change in the fluid medication in the reservoir. This change in pressure of the fluid medication in the reservoir creates a similar pressure change in the bio-compatible gel column, which is transferred to the sensing element of the pressure sensor mechanically. In various embodiments, the sensing element senses the change in pressure and generates a proportionate piezoelectric current which is fed to the ASIC of the pressure sensor which determines the pressure of the fluid medication in the reservoir and/or a change in the pressure of the fluid medication in the reservoir. In various embodiments, the ASIC outputs the pressure value and/or the change in pressure value to the processor of the medication delivery device to trigger an alarm if, for example, the pressure of the fluid medication in the reservoir exceeds a predetermined threshold or if the pressure of the fluid unexpectedly increases or unexpectedly decreases.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. A pressure sensor comprising:
a circuit board;
a sensing element eccentrically positioned on a first major side of the circuit board;
a cover portion mounted to the first major side of the circuit board and comprising a columnar portion defining a port, the columnar portion having a distal open end and a proximal open end that is adhered to the circuit board and that surrounds the sensing element such that the columnar portion is eccentrically positioned relative to the first major side of the circuit board; and
a column of bio-compatible gel disposed within the port;
wherein the columnar portion is adapted to be connected to a reservoir containing a fluid such that the fluid is exposed to the column of bio-compatible gel via the distal open end and such that a pressure of the fluid in the reservoir is translated by the column of bio-compatible gel to the sensing element such that the sensing element is adapted to detect the pressure of the fluid in the reservoir.

2. The pressure sensor of claim 1, wherein the columnar portion is positioned relative to the first major side of the circuit board such that the columnar portion is offset a first distance from a center of the first major side of the circuit board in a first direction and a second distance from the center of the first major side of the circuit board in a second direction.

3. The pressure sensor of claim 2, wherein the first distance and the second distance are substantially the same; and
wherein the first direction and the second direction are substantially perpendicular.

4. The pressure sensor of claim 1, wherein an inner surface of the port defines an angle change line;
wherein a distal portion of the inner surface of the port between the angle change line and the distal open end is cylindrical; and
wherein a proximal portion of the inner surface of the port between the angle change line and the proximal open end is angled inward.

5. The pressure sensor of claim 4, wherein the proximal portion of the inner surface of the port is angled inward at less than ten degrees.

6. The pressure sensor of claim 4, wherein the angle change line is located a distance from the circuit board that is equal to or greater than a distance from the circuit board of a furthest away portion of a wirebond attached to the sensing element.

7. The pressure sensor of claim 1, wherein an outer surface of the columnar portion of the cover portion comprises a distal circumferential shoulder adjacent the distal open end and a proximal circumferential shoulder spaced apart therefrom such that a circumferential groove is defined therebetween; and
wherein the circumferential groove is adapted to receive an O-ring to seal a connection between the reservoir and the columnar portion of the cover portion.

8. The pressure sensor of claim 1, further comprising an application specific integrated circuit (ASIC) positioned on the first major side of the circuit board; and.
wherein the cover portion further comprises an extended portion defining a chamber separated from the port;
wherein the ASIC is at least partially enclosed within the chamber of the extended portion;
wherein the ASIC receives a piezoelectric current from the sensing element which is proportional to the pressure of the fluid in the reservoir;
wherein the ASIC uses the received piezoelectric current to compute the pressure of the fluid in the reservoir and/or a change in the pressure of the fluid in the reservoir; and
wherein the ASIC is adapted to use the computed pressure of the fluid in the reservoir and/or the change in the pressure of the fluid in the reservoir to detect a reduced or stopped flow of the fluid from the reservoir.

9. The pressure sensor of claim 1, wherein the cover portion is adhered to the first major side of the circuit board using an adhesive;
wherein one or more surfaces of the cover portion in contact with the adhesive comprise one or more bumps projecting therefrom; and
wherein the one or more surfaces of the cover portion in contact with the adhesive have a rougher surface texture than other surfaces of the cover portion.

10. A method for measuring a pressure of a fluid in a reservoir, the method comprising:
connecting a pressure sensor to the reservoir, the pressure sensor comprising
a circuit board;
a sensing element eccentrically positioned on a first major side of the circuit board;
a cover portion mounted to the first major side of the circuit board and comprising a columnar portion defining a port, the columnar portion having a distal open end and a proximal open end that is adhered to the circuit board and that surrounds the sensing element such that the columnar portion is eccentrically positioned relative to the first major side of the circuit board; and
a column of bio-compatible gel disposed within the port;
wherein the columnar portion of the pressure sensor is connected to the reservoir such that the fluid in the reservoir is exposed to the column of bio-compatible gel via the distal open end; and
wherein the pressure of the fluid in the reservoir is translated by the column of bio-compatible gel to the sensing element such that the sensing element detects the pressure of the fluid in the reservoir.

11. The method of claim 10, wherein the columnar portion is positioned relative to the first major side of the circuit board such that the columnar portion is offset a first distance from a center of the first major side of the circuit board in a first direction and a second distance from the center of the first major side of the circuit board in a second direction.

12. The method of claim 10, wherein an inner surface of the port defines an angle change line;
wherein a distal portion of the inner surface of the port between the angle change line and the distal open end is cylindrical; and
wherein a proximal portion of the inner surface of the port between the angle change line and the proximal open end is angled inward.

13. The method of claim 12, wherein the proximal portion of the inner surface of the port is angled inward at less than ten degrees.

14. The method of claim 12, wherein the angle change line is located a distance from the circuit board that is equal to or greater than a distance from the circuit board of a furthest away portion of a wirebond attached to the sensing element.

15. A medication delivery device comprising:
a reservoir containing a fluid medication; and
a pressure sensor attached to the reservoir, the pressure sensor comprising:
a circuit board;
a sensing element eccentrically positioned on a first major side of the circuit board;
a cover portion mounted to the first major side of the circuit board and comprising a columnar portion defining a port, the columnar portion having a distal open end and a proximal open end that is adhered to the circuit board and that surrounds the sensing element such that the columnar portion is eccentrically positioned relative to the first major side of the circuit board; and
a column of bio-compatible gel disposed within the port;
wherein pressure sensor is attached to the reservoir via the columnar portion such that the fluid medication is exposed to the column of bio-compatible gel via the distal open end and such that a pressure of the fluid medication in the reservoir is translated by the column of bio-compatible gel to the sensing element such that the sensing element is adapted to detect the pressure of the fluid medication in the reservoir.
